# EUROPÄISCHE PATENTSCHRIFT

(11) **EP 0 617 939 B1**
(45) Veröffentlichungstag und Bekanntmachung des Hinweises auf die Patenterteilung: **02.12.1998**
(21) Anmeldenummer: 94105089.0
(22) Anmeldetag: 30.03.1994
(51) Int. Cl.: A61F 13/15

(54) **Wegwerfwindel**
Disposable diaper
Couche-culotte jetable

(30) Priorität: 30.03.1993 JP 71938/93
(43) Veröffentlichungstag der Anmeldung: 05.10.1994
(73) Patentinhaber: UNI-CHARM CORPORATION, Kawanoe-shi Ehime-ken (JP)
(72) Erfinder: Numano, Kazuki, Kawanoe-shi, Ehime-ken (JP); Sasaki, Tohru, Kawanoe-shi, Ehime-ken (JP)
(74) Vertreter: Sperling, Rüdiger, Dipl.-Ing.

(56) Entgegenhaltungen:
- FR-A- 2 668 364
- US-A- 4 897 084
- US-A- 5 069 678

## Beschreibung

### TECHNISCHER HINTERGRUND DER ERFINDUNG

Die vorliegende Erfindung betrifft eine Wegwerfwindel, die zum Aufsaugen und Aufnehmen von Urin und anderen körperlichen Ausscheidungen verwendet wird.

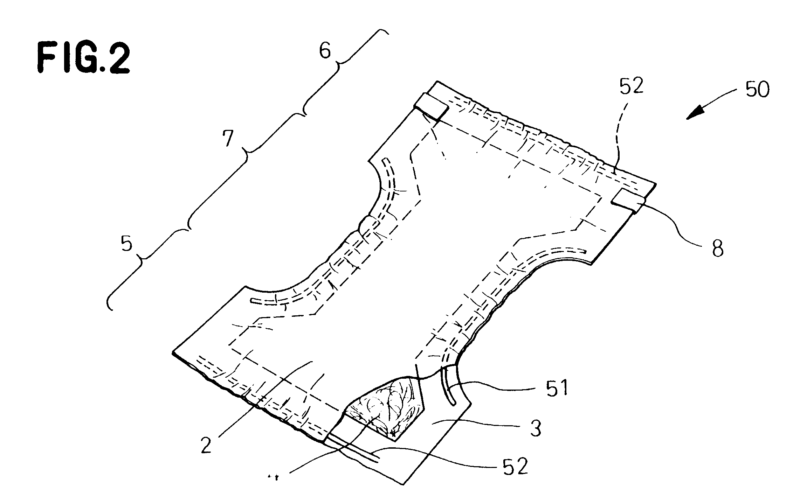

Fig. 2 der beiliegenden Zeichnungen ist eine perspektivische, teilweise ausgebrochene Darstellung, die als Beispiel eine herkömmliche Wegwerfwindel 50 zeigt. Die Windel 50 umfaßt eine flüssigkeitsdurchlässige Decklage 2, eine flüssigkeitsundurchlässige Außenlage 3, einen zwischen diese gelegten, flüssigkeitsabsorbierenden Kern 4, entlang dem rechten und dem linken Seitenrand eines Schrittbereiches vorgesehene, zur Begrenzung einer rechten und einer linken Beinöffnung bestimmte elastische Elemente 51 und entlang den in Längsrichtung einander gegenüberliegenden Enden des Vorder- und des Hinterteiles angeordnete elastische Elemente 52, die zur Begrenzung einer Hüftöffnung bestimmt sind. Der Hinterteil 6 ist entlang in Querrichtung einander gegenüberliegenden Seitenrändern mit Bandbefestigern 8 versehen, durch die der Hinterteil an dem Vorderteil 5 befestigt wird. Der Schrittbereich 7 ist eine in Längsrichtung zwischen dem Vorderteil und dem Hinterteil liegende Zone. Wegwerfwindeln des offenen Typs zeigen im allgemeinen einen derartigen Aufbau.

Ein Beispiel der herkömmlichen Wegwerfwindel ist in der japanischen Patentanmeldung Veröffentlichungsnummer 1992-289201 aufgezeigt. Genauer ist die in dieser Veröffentlichung offenbarte Windel eine Wegwerfwindel des Höschentyps, bei der elastische Elemente über einen breiten Bereich an einem Vorder- und einem Hinterteil angebracht sind, so daß sie sich auf eine Hüftlinie der Windel zu erstrecken, wodurch dieser Vorder- und Hinterteil über diesen breiten Bereich mit Elastizität versehen sind.

Ein weiters Beispiel zeigt die FR-A-2,668,364, die auch den Oberbegriff des Anspruchs 1 bildet.

Wenn die in Fig. 2 dargestellte herkömmliche Wegwerfwindel einem Träger angelegt wird, kommen die zur Begrenzung der Beinöffnungen bestimmten Seitenränder des Schrittbereiches und die zur Begrenzung der Hüftöffnung bestimmten, in Längsrichtung einander gegenüberliegenden Enden in dichten Kontakt mit der Haut des Trägers. Abschnitte des Vorder- und des Hinterteiles, die von den in Längsrichtung einander gegenüberliegenden Enden entfernt und nahe dem Schrittbereich liegen, kommen allgemein nur unter Schwierigkeiten in dichten Kontakt mit der Haut des Trägers, wodurch oftmals ein Passgefühl beeinträchtigt wird und ein rasches Aufsaugen des ausgeschiedenen Urins problematisch ist.

Zur Lösung dieses Problems wurde bereits eine Technik vorgeschlagen, gemäß welcher geeignetes elastisches Material für eine Decklage und/oder eine Außenlage der Windel verwendet wird. Derartiges Material ist jedoch relativ teuer und erhöht notwendigerweise die Materialkosten.

Die in der Veröffentlichung Nr. 1992-289201 offenbarte Technik beabsichtigt das Problem durch Verwendung relativ preiswerter Gummifäden zu lösen. Gemäß dieser Technik werden jedoch eine Vielzahl von Gummifäden an einer Windel in Querrichtung derselben angebracht, um so als ein elastisches Element für eine Hüftöffnung der Windel zu dienen, und eine Vielzahl von Gummifäden werden an der Windel in Längsrichtung derselben angebracht, um als das elastische Element für Beinöffnungen der Windel zu dienen. Das Anbringen der Gummifäden an der Windel in zwei Richtungen erfordert zwei getrennte Arbeitsschritte, was notwendigerweise eine Produktionslinie verlängert und die Anzahl der mechanischen Ausrüstungen erhöht. Somit hat eine derartige Technik in sich einen nachteiligen Faktor, der die Herstellungskosten der Windel erhöht.

Im Hinblick auf die vorstehend erwähnten Probleme ist es eine Hauptaufgabe der Erfindung, diese nach dem Stand der Technik ungelöst gebliebenen Probleme durch eine verbesserte Anordnung in der Weise zu lösen, daß preiswerte, gummiartige elastische Fäden umfassende elastische Elemente von den jeweiligen Beinöffnungen entlang einer gekrümmten Bahn über den Vorder- und den Hinterteil verlaufen, so daß das um die linke Beinöffnung vorgesehene elastische Element sich weiter zum rechten Seitenrand der Hüftöffnung erstreckt und das um die rechte Beinöffnung vorgesehene elastische Element sich weiter zum linken Seitenrand der Hüftöffnung erstreckt.

### KURZBESCHREIBUNG DER ERFINDUNG

Die vorstehend dargelegte Aufgabe wird gemäß der Erfindung durch eine Wegwerfwindel gelöst, umfassend eine flüssigkeitsdurchlässige Decklage, eine flüssigkeitsundurchlässige Außenlage, einen zwischen diese gelegten, flüssigkeitsabsorbierenden Kern und um eine Hüftöffnung bzw. Beinöffnungen jeweils vorgesehene elastische Elemente, wobie die um jede der Beinöffnungen angeordneten elastischen Elemente ein erstes elastisches Element und ein zweites elastisches Element umfassen, die eine Vielzahl von gummiartigen elastischen Fäden einschließen, die weiter parallel zueinander von in Querrichtung einander gegenüberliegenden Rändern eines Schrittbereiches in den Vorder- und den Hinterteil verlaufen, wobei das der linken Beinöffnung zugehörige erste elastische Element sich weiter entlang einer gekrümmten Bahn in den Vorder- und Hinterteil erstreckt und an einem Seitenrand des Vorder- und des Hinterteiles endet, während das einer zweiten Beinöffnung zugehörige zweite elastische Element sich weiter entlang einer gekrümmten Bahn in den Vorder- und den Hinterteil erstreckt und an dem anderen Seitenrand des Vorder- und des Hinterteiles endet.

Bei der Wegwerfwindel mit vorstehend beschriebenem Aufbau verläuft das um die erste Beinöffnung vorgesehene erste elastische Element weiter entlang der gekrümmten Bahn in jeden Teil zu einem Seitenrand des Vorder- und des Hinterteiles und das um die zweite Beinöffnung vorgesehene zweite elastische Element verläuft weiter entlang der gekrümmten Bahn in jeden Teil zum anderen Seitenrand des Vorder- und des Hinterteiles, so daß der Vorderteil und der Hinterteil in Richtung des Vorderteiles bzw. des Hinterteiles dehnbar sind. Folglich erlaubt es die erfindungsgemäße Windel nicht nur, den vorderen und hinteren Hüftbereich, sondern auch den Teil des Vorderteiles und des Hinterteiles, der sich von dem vorderen und dem hinteren Hüftbereich zum Schrittbereich erstreckt, zuverlässig in dichte Berührung mit der Haut des Trägers zu bringen, wenn die Windel an den Körper des Trägers angelegt ist.

### KURZE BESCHREIBUNG DER ZEICHNUNGEN

Nachfolgend wird eine erfindungsgemäße Wegwerfwindel im Detail unter Bezug auf die beiliegenden Zeichnungen beschrieben, wobei:
Fig. 1 eine perspektivische Darstellung ist, die teilweise ausgebrochen die Wegwerfwindel gemäß der Erfindung zeigt; und
Fig. 2 eine Fig. 1 ähnliche Ansicht ist, die jedoch die typische Wegwerfwindel nach dem Stand der Technik zeigt.

Es sei angemerkt, daß mit den Teilen der in Fig. 2 gezeigten herkömmlichen Windel gemeinsam vorliegende Teile durch gemeinsame Bezugszeichen bezeichnet sind.

### BEVORZUGTE AUSFÜHRUNGSFORMEN DER ERFINDUNG

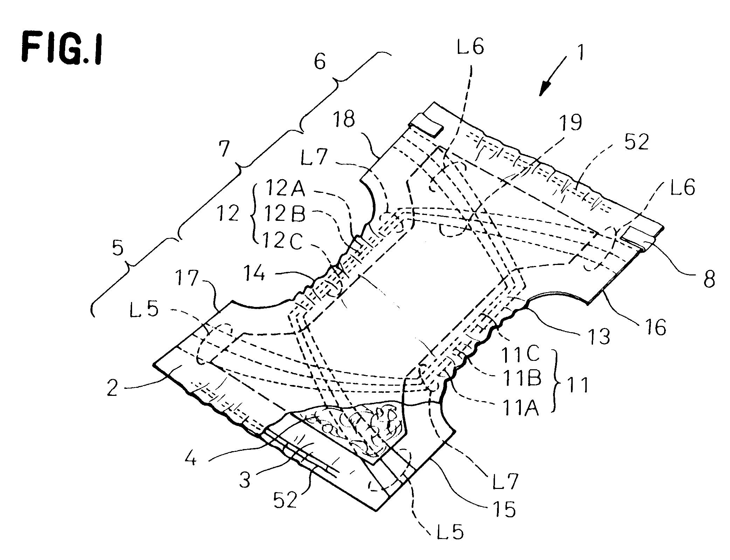

Fig. 1 zeigt in teilweise ausgebrochener perspektivischer Ansicht eine Wegwerfwindel 1 gemäß der Erfindung. Diese Windel 1 ist der in Fig. 2 dargestellten herkömmlichen Windel 50 insofern ähnlich, als diese Windel 1 ebenfalls eine flüssigkeitsdurchlässige Decklage 2, eine flüssigkeitsundurchlässige Außenlage 3, einen zwischen diese gelegten flüssigkeitsabsorbierenden Kern 4, einen Vorder- und einen Hinterteil 5, 6, deren in Längsrichtung äußere Enden mit elastischen Elementen 52 versehen sind, und am Hinterteil 6 an in Querrichtung einander gegenüberliegenden Seitenrändern 16, 18 in der Ebene der Hüftlinie der Windel 1 angebrachte Bandbefestiger 8 umfaßt, so daß diese Bestandteile nicht weiter im Detail beschrieben werden.

Die Windel 1 schließt elastische Elemente 11, 12 ein, die parallel mit den in Querrichtung einander gegenüberliegenden Seitenrändern 13, 14 des Schrittbereiches 7 verlaufend vorgesehen sind, die zur Begrenzung der jeweiligen Beinöffnungen bestimmt sind. Jedes der elastischen Elemente 11, 12 umfaßt eine Vielzahl von gummiähnlichen elastischen Fäden 11A, 11B, 11C oder 12A, 12B, 12C. Diese gummiähnlichen elastischen Fäden erstrecken sich weiter vom Schrittbereich 7 in den Vorder- und den Hinterteil 5, 6, wobei die gummiähnlichen elastischen Fäden 11A, 11B, 11C vom Umfang einer linken Beinöffnung der Windel 1 ausgehend an in Längsrichtung einander gegenüberliegenden Enden des Schrittbereiches gekrümmt sind, so daß sie in den Vorderteil und den Hinterteil 5, 6 verlaufen und am rechten Seitenrand 17, 18 dieser Teile enden, die zur Begrenzung eines Teiles einer Hüftöffnung der Windel 1 bestimmt sind, während die gummiähnlichen elastischen Fäden 12A, 12B, 12C ausgehend vom Umfang einer rechten Beinöffnung der Windel 1 in ähnlicher Weise gekrümmt sind, so daß sie in die jeweiligen Teile verlaufen und an den linken Seitenrändern 15, 16 derselben enden, die zur Begrenzung des anderen Teiles der Hüftöffnung bestimmt sind. Folglich überkreuzen die gummiähnlichen elastischen Fäden 11A, 11B, 11C die gummiähnlichen elastischen Fäden 12A, 12B, 12C in in Längsrichtung in der Mitte liegenden Bereichen des Vorder- bzw. des Hinterteiles 5, 6. Die gummiähnlichen elastischen Fäden werden in gespanntem Zustand mit der Innenfläche der Außenlage 3 verbunden und erstrecken sich im Schrittbereich 7 außerhalb der jeweiligen seitlichen Seitenränder des flüssigkeitsabsorbierenden Kerns 4 im wesentlichen parallel zueinander. Somit bilden Teile der gummiähnlichen elastischen Fäden 11A, 11B, 11C und 12A, 12B, 12C, die sich über den Schrittbereich 7 und den Vorder- und den Hinterteil 5, 6 erstrecken, eine schleifenförmige Dichtung 19, die einen Mittelbereich der Windel umgibt. Im Vorder- und im Hinterteil 5, 6 beschreiben die gummiähnlichen elastischen Fäden eine von den jeweiligen Beinöffnungen zu den jeweiligen Seitenrändern des Vorder- bzw. Hinterteiles verlaufende bergförmige Kurve und der Zwischenfadenabstand dieser gummiähnlichen elastischen Fäden ist mit der Annäherung an die jeweiligen Seitenränder des Vorder- und des Hinterteiles zunehmend erweitert. In Fig. 1 sind Teile der jeweiligen gummiähnlichen elastischen Fäden, die sich über den Schrittbereich 7 erstrecken, allgemein durch L7 bezeichnet, und Teile, die sich über den Vorderteil 5 und den Hinterteil 6 erstrecken, allgemein durch L5 bzw. L6 bezeichnet. Eine Längsspannung der Teile L7 ist höher eingestellt als diejenige der Teile L5, L6.

Wird die Windel 1 mit dem beschriebenen Aufbau an den Körper des Trägers angelegt, so kommt die Hüftöffnung in dichten Kontakt mit der Haut des Trägers unter der Wirkung der elastischen Elemente 52, die Beinöffnungen kommen ebenfalls in dichten Kontakt mit der Haut unter der Wirkung der Abschnitte L7 der gummiähnlichen elastischen Fäden, und der Vorderteil und der Hinterteil 5, 6 kommen mit der Haut unter der von den Abschnitten L5, L6 der gummiähnlichen elastischen Fäden zur Hüftlinie hin ausgeübten Kontraktionkraft mit der Haut in dichten Kontakt. Im Hinblick auf die Tatsache, daß der flüssigkeitsabsorbierende Kern 4 relativ starr ist, kann der Effekt dieser Kontraktionskraft durch eine Anordnung verbessert werden, daß der flüssigkeitsabsobierende Kern 4 nicht mit der Außenlage 3 im Vorderteil und im Hinterteil 5, 6 verbunden ist. Falls erforderlich, kann der flüssigkeitsabsorbierende Kern mit der Decklage 2 verbunden sein oder mit der Außenlage 3 nur im Schrittbereich 7 verbunden sein.

Die Decklage 2 der Windel 1 kann aus Vliesstoff oder offenporiger Kunststoffolie, die Außenlage 3 aus Kunststoffolie, beispielsweise aus Polyethylen hergestellt, und der flüssigkeitsabsorbierende Kern 4 kann beispielsweise aus einer Mischung aus Faserpulpe und hochabsorbierenden Polymerpulvern gebildet sein.

Die gummiähnlichen elastischen Fäden 11A, 11B, 11C und 12A, 12B, 12C können in einem gewünschten Muster beispielsweise unter Verwendung der in der japanischen Patentanmeldung Veröffentlichungsnummer 1992-317649 aufgezeigten Technik angeordnet werden. Gemäß dieser Technik kann der Betriebsablauf zur Anordnung der elastischen Elemente in der Weise, daß sie sich vom Umfang der Beinöffnungen zu den Seitenrändern der Hüftlinie erstrecken, durch eine relativ einfache Vorrichtung und in einem einzelnen Arbeitsschritt erzielt werden.

Bei der erfindungsgemäßen Wegwerfwindel verläuft das um die linke Beinöffnung vorgesehene elastische Element weiter zum rechten Seitenrand des Vorder- und des Hinterteiles und das um die rechte Beinöffnung vorgesehene elastische Element verläuft weiter zum linken Seitenrand des Vorder- und des Hinterteiles, so daß eine Kontraktionskraft über den Vorder- und den Hinterteil zu der vorderen und der hinteren Hüftlinie dieser Teile ausgeübt wird und dadurch diese Teile mit der Haut des Trägers in dichten Kontakt gebracht werden. Das hat zur Folge, daß diese Teile nicht aufgebauscht sind.

Relativ preiswerte, gummiähnliche elastische Fäden können als elastische Elemente verwendet werden und daher kann im Vergleich zu dem Fall, in dem dehnbares Material für die Deck- und die Außenlage verwendet wird, um dem Vorderteil und dem Hinterteil die gewünschte Elastizität zu verleihen, die Steigerung der Materialkosten verringert werden.

Die elastischen Elemente werden auf die Beinöffnungen wie auch auf den Vorder- und den Hinterteil in einem einzelnen Arbeitsschritt zugeliefert, so daß der Arbeitsvorgang vereinfacht werden kann und dadurch die Produktivität im Vergleich zu dem Fall, in dem die elastischen Elemente in getrennten Arbeitsschritten auf die Beinöffnungen und auf den Vorder- und den Hinterteil zugeliefert werden, verbessert werden kann.

## Patentansprüche

1. Wegwerfwindel, umfassend eine flüssigkeitsdurchlässige Decklage (2), eine flüssigkeitsundurchlässige Außenlage (3), einen zwischen diese gelegten, flüssigkeitsabsorbierenden Kern (4) und um eine Hüftöffnung bzw. Beinöffnungen jeweils vorgesehene elastische Elemente (11, 12, 52), wobei die um die Beinöffnungen angeordneten elastischen Elemente (11, 12) ein erstes elastisches Element (11) und ein zweites elastisches Element (12) umfassen, die eine Vielzahl von gummiartigen elastischen Fäden (11A, 11B, 11C; 12A, 12B, 12C) einschließen, die parallel zueinander von in Querrichtung einander gegenüberliegenden Rändern (13, 14) eines Schrittbereiches (7) entlang einer gekrümmten Bahn in den Vorder- (5) und den Hinterteil (6) verlaufen, **dadurch gekennzeichnet,** daß sich das der linken Beinöffnung zugehörige erste elastische Element (11) weiter in den Vorder- (5) und Hinterteil (6) erstreckt und an einem Seitenrand (17, 18) des Vorder- (5) und des Hinterteiles (6) endet, während sich das der zweiten Beinöffnung zugehörige zweite elastische Element (12) weiter in den Vorder- (5) und den Hinterteil (6) erstreckt und an dem anderen, entgegengesetzten Seitenrand (15, 16) des Vorder- (5) und des Hinterteiles (6) endet.

2. Wegwerfwindel gemäß Anspruch 1, wobei ein Zwischenfadenabstand der mehrfach vorliegenden gummiähnlichen elastischen Fäden (11A, 11B, 11C; 12A, 12B, 12C) mit Annäherung an die jeweiligen Seitenränder (15, 16, 17, 18) der Vorder- (5) und des Hinterteiles (6) zunehmend erweitert ist.

3. Wegwerfwindel gemäß Anspruch 1, wobei das erste elastische Element (11) das zweite elastische Element (12) in in Längsrichtung mittleren Bereichen des Vorder- (5) und des Hinterteiles (6) überkreuzt und dadurch das erste und das zweite elastische Element (11, 12) eine einen Mittelbereich der Windel umgebende, schleifenförmige Dichtung bilden.

4. Wegwerfwindel gemäß Anspruch 1, wobei eine Längsspannung von über den Schrittbereich (7) verlaufenden Abschnitten so eingestellt ist, daß sie höher ist als diejenige von Abschnitten, die sich über den Vorder- (5) und den Hinterteil (6) erstrecken.

## Claims

1. A disposable diaper comprising a liquid-permeable topsheet (2), a liquid-impermeable backsheet (3), a liquid-absorbent core (4) sandwiched therebetween, and elastic members (11, 12, 52) provided around a waist-opening and leg-openings, respectively, wherein the elastic members (11, 12) provided around said leg-openings comprise a first elastic member (11) and a second elastic member (12) including a plurality of rubber-like elastic threads (11A, 11B, 11C; 12A, 12B, 12C) extending in parallel with one another from transversely opposite side edges (13, 14) of a crotch zone (7) along a curved path into the front (5) and rear bodies (6), **characterized in that** the first elastic member (11) associated with the left leg-opening further extends into the front (5) and rear bodies (6) and terminates at a side edge (17, 18) of the front (5) and rear bodies (6) while the second elastic member (12) associated with a second leg-opening further extends into the front (5) and rear bodies (6) and terminates at the other opposite side edge (15, 16) of the front (5) and rear bodies (6).

2. A disposable diaper according to Claim 1, wherein an interthread spacing of said plural rubber-like elastic threads (11A, 11B, 11C; 12A, 12B, 12C) is progressively widened as they get near the respective side edges (15, 16, 17, 18) of the front (5) and rear bodies (6).

3. A disposable diaper according to Claim 1, wherein said first elastic member (11) crosses said second elastic member (12) on longitudinal center areas of said front (5) and rear bodies (6) and thereby said first and second elastic members (11, 12) define a loop-shaped seal surrounding a central zone of said diaper.

4. A disposable diaper according to Claim 1, wherein an elongation stress of portions extending over said crotch zone (7) is adjusted to be higher than those of portions extending over said front (5) and rear bodies (6).

## Revendications

1. Lange à usage unique, comprenant une couche de revêtement (2) perméable aux liquides, une couche extérieure (3) imperméable aux liquides, un corps central (4) absorbant les liquides, inséré entre ces deux couches, et des éléments élastiques (11, 12, 52) respectivement prévus autour d'une ouverture de passage de hanches et d'ouvertures de passage des jambes, les éléments élastiques (11, 12) disposés autour des ouvertures de passage des jambes comprenant un premier élément élastique (11) et un second élément élastique (12) englobant une multiplicité de fils élastiques (11A, 11B, 11C; 12A, 12B, 12C) du type caoutchouc qui évoluent parallèlement entre eux depuis des bords (13, 14) d'une zone d'entrejambe (7), mutuellement opposés en sens transversal, en suivant une trajectoire incurvée traversant la partie avant (5) et la partie arrière (6), caractérisé en ce que le premier élément élastique (11) associé à l'ouverture de passage des jambes de gauche se prolonge dans les parties avant (5) et arrière (6) et se termine au niveau du bord latéral opposé (17, 18) de la partie avant (5) et de la partie arrière (6), respectivement, tandis que le second élément élastique (12) associé à la seconde ouverture de passage de jambes se prolonge dans les parties avant (5) et arrière (6), pour s'arrêter au niveau du bord latéral (15, 16) opposé des parties avant (5) et arrière (6), respectivement.

2. Lange à usage unique selon la revendication 1, dans lequel l'espace entre-fil de la pluralité de fils élastiques (11A, 11B, 11C; 12A, 12B, 12C) du type caoutchouc s'élargit progressivement à mesure qu'il se rapproche des bords latéraux respectifs (15, 16, 17, 18) de la partie avant (5) et de la partie arrière (6).

3. Lange à usage unique selon la revendication 1, dans lequel le premier élément élastique (11) croise le second élément élastique (12) dans des régions longitudinalement médianes des parties avant (5) et arrière (6) et, de ce fait, le premier et le second élément élastique (11, 12) donnent naissance à une garniture d'étanchéité formant une boucle qui entoure une région médiane du lange.

4. Lange à usage unique selon la revendication 1, dans lequel une tension longitudinale de portions qui s'étendent sur la zone d'entrejambe (7) est ajustée pour être supérieure à celle de portions qui s'étendent sur les parties avant (5) et arrière (6).
